# FASCICULE DE BREVET EUROPEEN

(11) **EP 3 006 810 B1**
(45) Date de publication et mention de la délivrance du brevet: **15.05.2019**
(21) Numéro de dépôt: 15187940.0
(22) Date de dépôt: 16.02.2015
(51) Int. Cl.: F17C 13/06

(54) **CAPOTAGE DE PROTECTION AVEC SYSTÈME D ACCROCHAGE PIVOTANT POUR BOUTEILLE DE GAZ**
SCHUTZHAUBE MIT SCHWENKBAREM EINRASTSYSTEM FÜR GASFLASCHE
PROTECTIVE HOOD WITH PIVOTING ATTACHMENT SYSTEM FOR A GAS CYLINDER

(30) Priorité: 12.03.2014 FR 1452039
(43) Date de publication de la demande: 13.04.2016
(62) Demande divisionnaire de: 15305224.6
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: FRENAL, Antoine, 95460 EZANVILLE (FR); GRAVIERE, Vincent, 94200 Ivry Sur Seine (FR); LIGONESCHE, Renaud, 95220 HERBLAY (FR); TARANTELLO, Chiara, 92800 PUTEAUX (FR); TREVISAN, Adrien, 92200 NEUILLY SUR SEINE (FR); VIGNEROL, Samuel, 95380 LOUVRES (FR)
(74) Mandataire: Pittis, Olivier

(56) Documents cités:
- EP-A1- 0 629 812
- EP-A1- 2 330 335
- EP-A1- 2 586 481
- DE-A1- 10 057 469
- FR-A1- 2 976 048
- GB-A- 2 246 596
- US-A1- 2009 272 443

## Description

La présente invention concerne un récipient de gaz muni d'un capotage de protection, encore appelé chapeau, telle une bouteille de gaz, en particulier de gaz médical, équipé d'un dispositif d'accrochage sur lits, brancards, fauteuils ou autres, venant épouser les contours extérieurs du corps de capotage lorsqu'il est replié dans sa position de repos.

Les gaz industriels et médicaux sont couramment conditionnés dans des récipients de gaz, typiquement des bouteilles de gaz, équipés d'un bloc robinet, avec ou sans détendeur intégré, à savoir un robinet simple de type ouvert/fermé ou un robinet à détendeur intégré, encore appelé RDI, permettant de contrôler débit et pression du gaz délivré.

Afin de protéger ce bloc robinet, il est courant agencer au niveau du col de la bouteille de gaz et autour dudit bloc robinet, un capotage de protection formant coque protectrice autour dudit bloc robinet. Un tel capotage est couramment appelé « chapeau ».

Par ailleurs, pour permettre d'accrocher l'ensemble bouteille/chapeau/robinet à un support, tel un barreau de lit d'hôpital, un brancard, une tringle ou support d'accrochage d'un véhicule d'urgence, par exemple une unité de SAMU ou analogue, il est usuel d'aménager sur le chapeau, un système d'accrochage fixe.

En fait, il existe deux types de systèmes d'accrochage, à savoir ceux fixes et ceux mobiles.

A titre d'exemple de système d'accrochage fixe, on peut citer les encoches, crochets ou analogues aménagés portés par le capotage, soit lors de la fabrication du corps du capotage lui-même et qui en font intégralement partie, soit qui sont fixés au corps de capotage après coup.

A titre d'exemple de système d'accrochage mobile, on peut citer les supports pivotants ou translatifs venant se fixer à la face arrière ou à une face latérale du capotage.

Des capotages pour bouteilles de gaz à système d'accrochage fixe ou mobile sont décrits par les documents EP-A-629812, DE-A-10057469, US-A-2004/020793 et EP-A-2586481. Par ailleurs, le document EP-A-3114388, qui fait partie de l'état de la technique selon l'Article 54(3) CBE, divulgue un capotage de protection à dispositif d'accrochage pivotant.

Toutefois, ces différents capotages et systèmes d'accrochage existants présentent des inconvénients.

Ainsi, beaucoup de systèmes d'accrochage s'étendent au-delà du corps de la bouteille, même lorsqu'ils sont repliés dans leur position de repos, ce qui conduit à augmenter l'encombrement important de l'ensemble bouteille/capotage.

Or, augmenter l'encombrement de l'ensemble engendre, de façon aisément compréhensible, des problèmes de stockage des bouteilles ainsi équipées, en particulier de rangement dans les racks ou autres paniers servant à leur transport groupé.

De plus, le fait que beaucoup dépassent de systèmes d'accrochage pivotants du diamètre général de la bouteille, même repliés, est une source d'accidents pour les utilisateurs dont les vêtements par exemple peuvent s'accrocher par inadvertance au système et engendrer une chute sur le sol de la bouteille, avec un risque de blessure pour les personnels à proximité.

Ainsi, le document EP-A-2586481 enseigne un système d'accrochage pivotant pour chapeau de bouteille de gaz. Ce système est formé de deux bras pivotants qui sont reliés l'un à l'autre par une tige rigide. En position repliée, la tige vient buter sur le corps du chapeau en restant tangentielle à celui-ci, alors que les deux bras viennent se placer de part et d'autre du haut du corps de la bouteille. Un tel système présente donc les inconvénients susmentionnés.

D'autres sont d'architecture compliquée et/ou sont peu pratiques à manipuler.

Il existe aussi un capotage possédant un logement spécifique dans lequel vient s'insérer un bras pivotant. Si cela permet d'éviter que le bras ne déborde du corps du capotage, lorsqu'il est replié, cela a aussi comme inconvénient majeur de rendre le nettoyage les parties intérieures du capotage et du dispositif d'accrochage plus compliqué et d'engendrer une accumulation inévitable de poussières et autres saletés au fil du temps dans ces régions du capotage, c'est-à-dire un état de propreté peu compatible avec une utilisation en domaine hospitalier. En outre, la manipulation de ce bras pivotant n'est pas forcément aisée pour l'utilisateur qui peut se coincer les doigts lors de son déploiement et de l'accrochage de l'ensemble à un support, tel un barreau de lit d'hôpital.

Au vu de cela, le problème qui se pose est de proposer un récipient de gaz muni d'un capotage de protection ou chapeau comprenant un dispositif d'accrochage amélioré ne présentant pas les inconvénients susmentionnés.

La solution de l'invention est un récipient de gaz équipé d'un bloc robinet avec ou sans détendeur intégré, comprenant un capotage de protection, ou « chapeau », agencé autour d'au moins une partie dudit bloc robinet, ledit bloc robinet comprenant un raccord de remplissage situé en face arrière, servant à introduire du gaz dans le récipient et ledit capotage de protection comprenant :
- un corps de capotage formant coque protectrice délimitant un volume interne conçu pour recevoir tout ou partie du bloc robinet, et
- un dispositif d'accrochage pivotant entre plusieurs positions angulaires comprenant :
   - une position de repos dans laquelle le dispositif d'accrochage est totalement replié et
   - une position d'accrochage dans laquelle le dispositif d'accrochage est totalement déplié, et dans lequel
- le dispositif d'accrochage comprend une structure tridimensionnelle comprenant deux bras portant chacun une encoche de fixation à un support, ladite structure tridimensionnelle comprenant une face intérieure conformée pour venir épouser le profil extérieur du corps de capotage, lorsque le dispositif d'accrochage est en position de repos, , c'est-à-dire qu'il est complètement replié et positionné autour du capotage, et
- la structure tridimensionnelle se positionne face à au raccord de remplissage, lorsque ladite structure tridimensionnelle est en position de repos et repliée contre le corps du capotage.

Selon le cas, le récipient selon l'invention peut comprendre l'une ou plusieurs des caractéristiques techniques suivantes :
- le dispositif d'accrochage est fixé de manière pivotante autour d'un axe AA situé sur la face arrière du corps du capotage.
- la face arrière et/ou les faces latérales du corps de capotage est/sont conformées pour présenter un profil extérieur complémentaire de la face intérieure de la structure tridimensionnelle.
- la face arrière et/ou les faces latérales du corps de capotage présentent au moins une empreinte réalisée dans la coque externe du corps dans laquelle vient se loger au moins une partie de la structure tridimensionnelle en épousant le profil extérieur de ladite au moins une empreinte.
- la face intérieure de la structure tridimensionnelle est incurvée.
- la structure tridimensionnelle a une forme générale incurvée.
- le capotage comprend un système de blocage permettant de bloquer et maintenir le dispositif d'accrochage en position de repos.
- le dispositif d'accrochage pivote entre la position de repos et la position d'accrochage d'un angle (α) inférieur à 180°, typiquement compris entre 90° et 170°, de préférence d'au moins 110°.
- le capotage comprend au moins un montant-support solidaire du corps de capotage et portant une poignée de portage.
- le corps du capotage et la structure tridimensionnelle sont formés de matériau polymère. Typiquement, le matériau de type polymère est un matériau plastique, tel que PVC, PE, PET, PP, PMMA, PU, PA...
- les deux bras sont reliés l'un à l'autre par au moins un élément de jonction, de préférence les deux bras et ledit au moins un élément de jonction sont formés d'une seule pièce, notamment par moulage.
- le capotage comprend un système de blocage permettant de bloquer et maintenir le dispositif d'accrochage en position de repos.
- il est équipé d'un bloc robinet,avec ou sans détendeur intégré..
- il est choisi parmi les bouteilles de gaz.
- il est une bouteille de gaz ayant une taille comprise entre 10 et 150 cm.
- il est une bouteille de gaz contenant de 0,5 à 20 litres (contenance en équivalent eau).
- il est une bouteille de gaz de corps cylindrique creux.
- il est une bouteille de gaz comprenant un col portant un orifice de sortie du gaz.
- il est une bouteille de gaz contenant un gaz ou mélange gazeux choisi parmi l'oxygène, l'air, un mélange N₂O/O₂, un mélange He/O₂, un mélange NO/azote ou tout autre gaz ou mélange gazeux.
- le bloc robinet est de type à détendeur intégré, c'est-à-dire un RDI.
- la bouteille est en acier, en un alliage d'aluminium ou en matériau composite.
- la bouteille contient un gaz à une pression allant jusqu'à 350 bar environ.
- un couvercle protecteur situé dans l'ouverture ou espacement entre les deux bras de la structure tridimensionnelle vient recouvrir le raccord de remplissage lorsque la structure tridimensionnelle est repliée en position de repos.

L'invention concerne aussi une utilisation d'un récipient de gaz selon l'invention pour distribuer un gaz ou mélange gazeux, en particulier un gaz ou mélange gazeux est choisi parmi l'oxygène, air, N₂O/O₂, He/O₂ et NO/azote.

L'invention va maintenant être mieux comprise grâce à la description détaillée suivante, faite à titre illustratif mais non limitatif, en référence aux figures annexées parmi lesquelles :
- la Figure 1 représente une vue de la face avant d'un capotage de protection équipant une bouteille de gaz selon l'invention,
- la Figure 2 représente une vue de la face arrière du capotage de la Figure 1,
- la Figure 3 est une vue de ¾ arrière du capotage de la Figure 1, et
- la Figure 4 représente un ensemble formé d'une bouteille de gaz équipée du capotage de la Figure 1 vu en position couchée sur une surface.

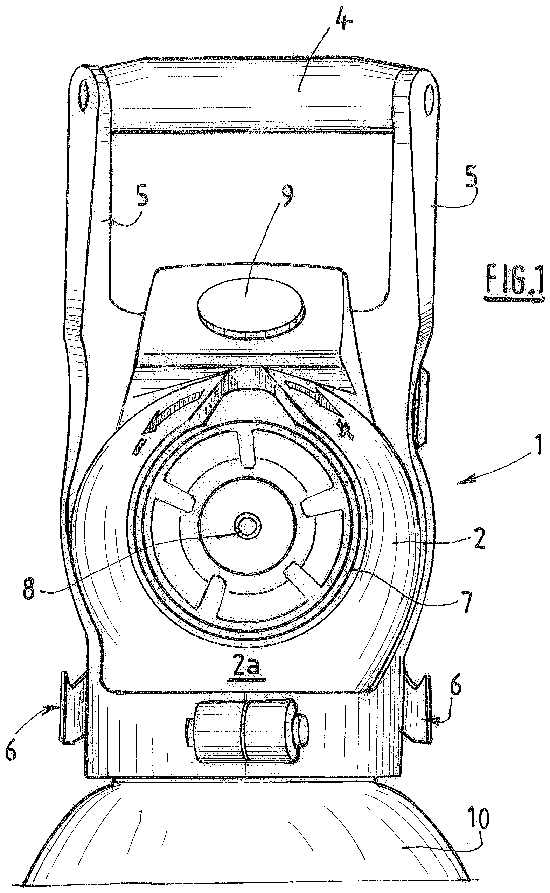

Les Figures 1 à 4 présentent un mode de réalisation d'un capotage de protection 1 rigide, couramment appelé « chapeau », agencé autour d'un bloc robinet, à savoir un robinet avec ou sans détendeur intégré, lui-même fixé sur le col d'une bouteille de gaz 10 selon l'invention.

La bouteille de gaz 10 a typiquement une taille entre 10 et 150 cm, et une contenance de 0,5 à 20 litres (en équivalent eau).

Le capotage de protection 1 sert à protéger le bloc robinet contre les chocs, que le dit bloc robinet soit du type avec détendeur intégré ou RDI, ou du type sans détendeur intégré.

A ce titre, le capotage de protection 1 comprend un corps 2 de capotage formant une coque protectrice autour d'un volume interne dimensionné pour recevoir le bloc robinet, une poignée de portage 4 conçue pour être prise en main par un opérateur, ladite poignée de portage 4 étant formée d'un matériau rigide, et portée par un ou plusieurs montants-supports 5 reliant mécaniquement le corps 2 de capotage à la poignée de portage 4, c'est-à-dire que le ou les montants-supports 5 sont solidarisés à la poignée de portage 4 de manière à permettre à un utilisateur de transporter facilement l'ensemble chapeau/robinet/bouteille.

Le corps 2 du capotage 1 est typiquement en un matériau de type polymère et/ou métal, préférentiellement en matériau plastique, tel que PVC, PE, PET, PP, PMMA, PU, PA... De même, les montants-supports 5 peuvent être formés d'un matériau plastique, comme le corps du capotage 1, mais aussi en alliage d'aluminium ou tout autre matériau métallique.

Le capotage de protection 1 présente par ailleurs des ouvertures donnant accès au bloc robinet situé dans le volume interne du corps 2 de capotage 1, en particulier aux raccords, volant rotatif...

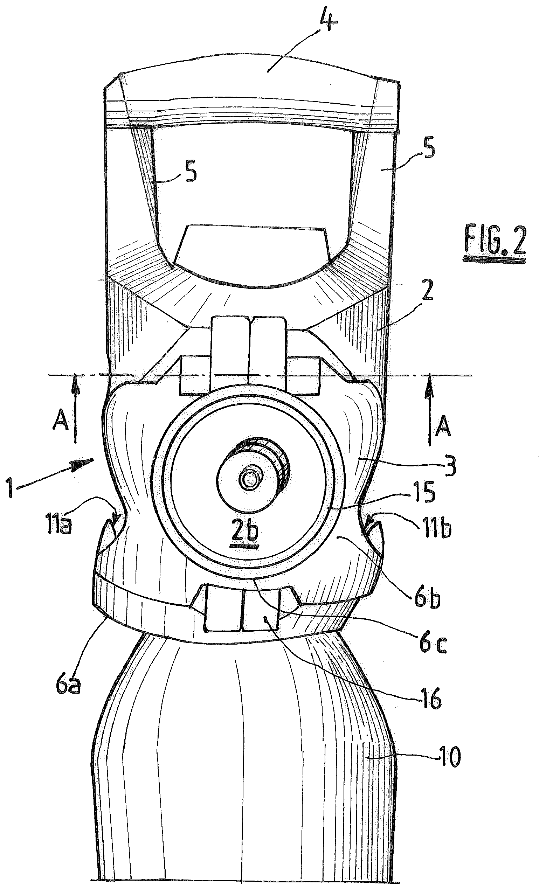

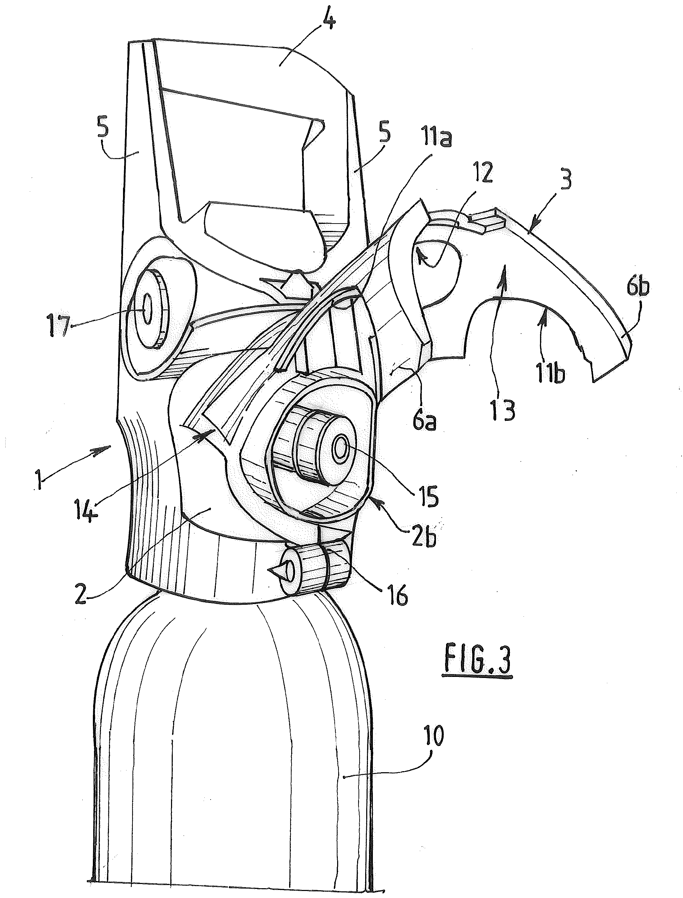

En effet, le bloc robinet, typiquement un robinet de type RDI, comprend, comme illustré en Figure 3, un volant rotatif 7, situé en face avant 2a, manipulable par un utilisateur pour contrôler le débit de gaz, un raccord de sortie de gaz 8 pour soutirer le gaz stocké dans la bouteille 10, un manomètre 9 pour contrôler la pression du gaz, un raccord de remplissage 15 (Fig.2 et 3), situé en face arrière 2b, servant à introduire du gaz dans la bouteille 10 lorsque celle-ci est vide, et un raccord de sortie de gaz sous pression 17

L'assemblage du capotage de protection 1 et sa fixation autour du bloc de robinet porté par la bouteille de gaz 10, se fait par vissage au moyen d'éléments de fixation, à savoir des vis ou analogues.

Afin de permettre l'accroche ou l'arrimage de l'ensemble bouteille/bloc robinet/capotage à un support, tel un barreau de lit d'hôpital ou de brancard, le capotage de protection 1 comprend, du côté de sa face arrière 2b, un dispositif d'accrochage 3 pivotant autour d'un axe AA, entre une position totalement repliée, dite « de repos », c'est-à-dire la position adoptée par le dispositif d'accrochage 3 lorsqu'il est rangé et en contact ou quasi-contact du corps 2 du capotage 1, et une position totalement dépliée dite « d'accrochage », c'est-à-dire la position adoptée par le dispositif d'accrochage 3 lorsqu'il est complètement sorti et peut être accroché à un support, tel un barreau de lit ou analogue.

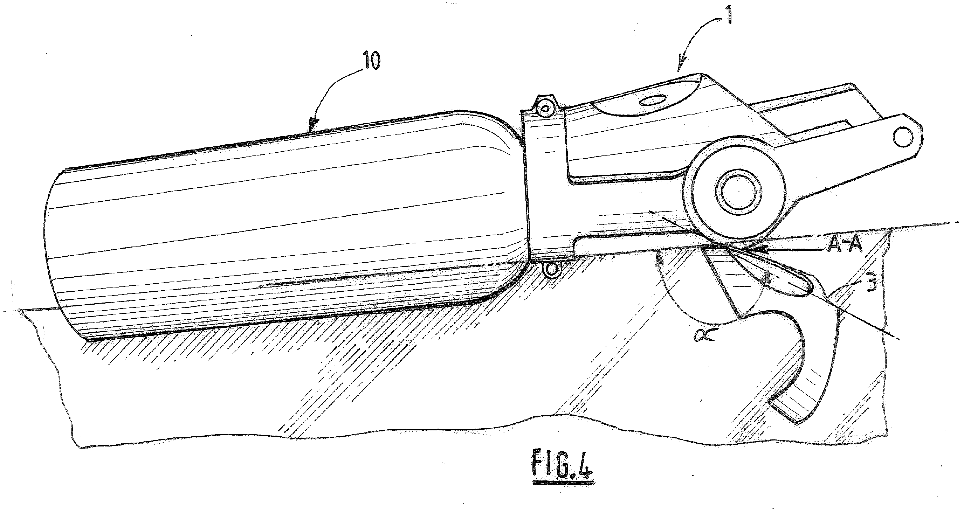

Ainsi, la Figure 2 montre le dispositif d'accrochage 3 en position de repos, c'est-à-dire replié, alors que les Figures 3 et 4 montrent le dispositif d'accrochage 3 en position d'accrochage, c'est-à-dire totalement déplié.

Ces deux positions constituent donc les positions angulaires extrêmes que peut adopter le dispositif d'accrochage 3 lors de son pivotement autour de l'axe AA.

Typiquement, l'angle α de pivotement entre ces deux positions angulaires extrêmes est inférieur ou égale à 170° et supérieur à 0°, préférentiellement supérieur à 90°, par exemple un angle de l'ordre de 150°.

Afin de résoudre certains des problèmes posés par les dispositifs d'accrochage connus, dans le cadre de la présente invention, on veille à une parfaite intégration du dispositif d'accrochage 3 autour du corps 2 du capotage de protection 1.

Pour ce faire, le dispositif d'accrochage 3 selon l'invention forme une structure tridimensionnelle incurvée 3, 6a, 6b, 11a, 11b venant épouser les contours du corps 2 de capotage, lorsque le dispositif d'accrochage 3 est en position de repos, c'est-à-dire replié comme illustré en figure 2.

Plus précisément, la structure tridimensionnelle incurvée 3, 6a, 6b, 11a, 11b comprend deux bras 6a, 6b, à savoir un bras droit 6a et un bras gauche 6b, venant prendre le corps 2 de capotage en sandwich, lorsque le dispositif d'accrochage 3 est en position de repos.

Chacun des bras 6a, 6b porte une encoche de fixation 11a, 11b servant à l'accrochage de l'ensemble à un support, tel un barreau de lit d'hôpital ou analogue. Chaque encoche de fixation 11a, 11b a une forme arquée, par exemple conformée en arc de cercle, de manière à épouser au mieux la forme d'un barreau tubulaire de lit, de brancard, de fauteuil roulant ou autre.

En d'autres termes, les encoches de fixation 11a, 11b des bras 6a, 6b sont conformées et dimensionnées pour permettre un arrimage d'un récipient de gaz équipé d'un capotage selon l'invention, à un barreau de lit hospitalier ou analogue, c'est-à-dire à une structure tubulaire.

La structure tridimensionnelle 3, 6a, 6b, 11a, 11b comprend, quant à elle, une face intérieure 13 conformée pour venir épouser le profil extérieur 14, c'est-à-dire la surface ou contour externe, du corps 2 de capotage 1 de manière à minimiser l'encombrement général du capotage 1, lorsque le dispositif d'accrochage 3 est replié comme sur la figure 2.

D'une façon générale selon l'invention, la face intérieure 13 de la structure tridimensionnelle, c'est-à-dire la surface ou paroi 13 de la structure tridimensionnelle 3, 6a, 6b, 11a, 11b faisant face au capotage, en particulier celle des deux bras 6a, 6b, est conformée de manière à venir épouser la forme ou le profil externe 14 du corps 2 de capotage 1 de sorte de minimiser l'encombrement global de la structure tridimensionnelle 3, 6a, 6b, 11a, 11b, lorsqu'elle est repliée, et donc éviter tout ou partie des inconvénients des capotages de l'art antérieur.

Ainsi, comme visible sur la Figure 3, la face 13 vient parfaitement épouser le contour extérieur de la face arrière 2b du capotage 1 en venant s'insérer dans l'empreinte 14 aménagée dans le corps 2 du capotage 1, laquelle empreinte 14 a une forme complémentaire de celle de la structure tridimensionnelle 3, 6a, 6b, 11a, 11b, lorsque la structure tridimensionnelle 3, 6a, 6b, 11a, 11b est dans sa position de repos ou totalement repliée, comme montré en Figure 2.

En d'autres termes, afin d'accroître l'intégration de la structure tridimensionnelle 3, 6a, 6b, 11a, 11b dans le corps 2 du capotage 1, on prévoit d'aménager sur le contour du corps 2 de capotage 1, au moins une empreinte 14 spécifique qui soit adaptée et conformée pour recevoir tout ou partie de la structure tridimensionnelle 3, 6a, 6b, 11a, 11b qui vient alors s'y loger en position repliée, c'est-à-dire de repos.

Ainsi, lorsque la structure tridimensionnelle 3, 6a, 6b, 11a, 11b est repliée, elle ne fait pas ou quasiment pas saillie extérieurement et n'augmente pas l'encombrement global du capotage 1.

La structure tridimensionnelle 3, 6a, 6b, 11a, 11b, outre l'accroche à un support tel un barreau de lit d'hôpital ou analogue, a une autre fonction qui est de stabiliser la bouteille 10 en position horizontale pour éviter qu'elle ne roule sur le sol ou sur toute autre surface. Pour ce faire, la structure tridimensionnelle 3, 6a, 6b, 11a, 11b, une fois totalement dépliée donc dans sa position d'accroche, peut servir de béquilles à la bouteille 10 lorsqu'elle est couchée à l'horizontal, comme illustré en Figure 4.

Par ailleurs, dans une variante de réalisation, la structure tridimensionnelle 3, 6a, 6b, 11a, 11b est conformée de manière à permettre une protection du raccord de remplissage 15 de la poussière, des intempéries, des mauvais usages...

A cette fin, l'ouverture ou espacement 12 entre les deux bras 6a, 6b peut être comblé et former un couvercle protecteur venant recouvrir le raccord de remplissage 15 situé sur la face arrière 2b du corps 2, lorsque la structure tridimensionnelle 3, 6a, 6b, 11a, 11b est en position de repos, c'est-à-dire repliée contre le corps 2 du capotage 1.

Le récipient de l'invention est particulièrement bien adapté à une utilisation dans le domaine de la santé, pour distribuer un gaz ou mélange gazeux médical.

## Revendications

1. Récipient de gaz (10) équipé d'un bloc robinet avec ou sans détendeur intégré, comprenant un capotage (1) de protection agencé autour d'au moins une partie dudit bloc robinet, ledit bloc robinet comprenant un raccord de remplissage (15) situé en face arrière et servant à introduire du gaz dans le récipient (10) et ledit capotage (1) de protection comprenant :
- un corps de capotage (2) formant coque protectrice délimitant un volume interne conçu pour recevoir tout ou partie du bloc robinet, et
- un dispositif d'accrochage (3) pivotant entre plusieurs positions angulaires comprenant :
. une position de repos dans laquelle le dispositif d'accrochage (3) est totalement replié et
. une position d'accrochage dans laquelle le dispositif d'accrochage (3) est totalement déplié, et dans lequel
- le dispositif d'accrochage (3) comprend une structure tridimensionnelle (3, 6a, 6b, 11a, 11b) comprenant deux bras (6a, 6b) portant chacun une encoche (11a, 11b) de fixation à un support, ladite structure tridimensionnelle (3, 6a, 6b, 11a, 11b) comprenant une face intérieure (13) conformée pour venir épouser le profil extérieur (14) du corps (2) de capotage (1), lorsque le dispositif d'accrochage (3) est en position de repos, et
- la structure tridimensionnelle (3, 6a, 6b, 11a, 11b) se positionne face au raccord de remplissage (15), lorsque ladite structure tridimensionnelle (3, 6a, 6b, 11a, 11b) est en position de repos et repliée contre le corps (2) du capotage (1).

2. Récipient selon la revendication précédente, **caractérisé en ce que** le dispositif d'accrochage (3) est fixé de manière pivotante autour d'un axe AA situé sur la face arrière (2b) du corps (2) du capotage (1).

3. Récipient selon l'une des revendications précédentes, **caractérisé en ce que** la face arrière (2b) et/ou les faces latérales du corps (2) de capotage (1) sont conformées pour présenter un profil extérieur complémentaire de la face intérieure (13) de la structure tridimensionnelle (3, 6a, 6b, 11a, 11b).

4. Récipient selon l'une des revendications précédentes, **caractérisé en ce que** la face arrière (2b) et/ou les faces latérales du corps (2) de capotage (1) présentent au moins une empreinte (14) réalisée dans la coque externe du corps (2) dans laquelle vient se loger au moins une partie de la structure tridimensionnelle (3, 6a, 6b, 11a, 11b) en épousant le profil extérieur de ladite au moins une empreinte (14).

5. Récipient selon la revendication 1, **caractérisé en ce que** la structure tridimensionnelle (3, 6a, 6b, 11a, 11b) comprend au moins une face intérieure (13) incurvée.

6. Récipient selon la revendication 1, **caractérisé en ce que** le dispositif d'accrochage (3) pivote entre la position de repos et la position d'accrochage d'un angle (α) inférieur à 180°, typiquement compris entre 90° et 170°.

7. Récipient selon la revendication 1, **caractérisé en ce qu'**il comprend au moins un montant-support (5) solidaire du corps de capotage (2) et portant une poignée de portage (4).

8. Récipient selon la revendication 1, **caractérisé en ce que** le corps (4) du capotage (1) et la structure tridimensionnelle (3, 6a, 6b, 11a, 11b) sont formés de matériau polymère.

9. Récipient selon la revendication 1, **caractérisé en ce que** les deux bras (6a, 6b) sont reliés l'un à l'autre par au moins un élément de jonction (6c), de préférence les deux bras (6a, 6b) et ledit au moins un élément de jonction (6c) sont formés d'une seule pièce, notamment par moulage.

10. Récipient selon la revendication 1, **caractérisé en ce qu'**il comprend un système de blocage (16) permettant de bloquer et maintenir le dispositif d'accrochage (3) en position de repos.

11. Récipient selon la revendication 1, **caractérisé en ce que** la structure tridimensionnelle (3, 6a, 6b, 11a, 11b) est conformée de manière à permettre une protection du raccord de remplissage (15), un couvercle protecteur étant situé dans l'ouverture ou espacement (12) entre les deux bras (6a,6b) de la structure tridimensionnelle (3, 6a, 6b, 11a, 11b) et venant recouvrir le raccord de remplissage (15), lorsque la structure tridimensionnelle (3, 6a, 6b, 11a, 11b) est repliée en position de repos.

12. Récipient selon la revendication 1, **caractérisé en ce qu'**il est choisi parmi les bouteilles de gaz, de préférence une bouteille de gaz (10) ayant une taille comprise entre 10 et 150 cm, et contenant de 0,5 à 20 litres (contenance en équivalent eau).

13. Utilisation d'un récipient de gaz selon l'une des revendications précédentes pour distribuer un gaz ou mélange gazeux.

14. Utilisation selon la revendication 13, **caractérisé en ce que** le gaz ou mélange gazeux est choisi parmi l'oxygène, air, N₂O/O₂, He/O₂ et NO/azote.

## Patentansprüche

1. Gasbehälter (10), der mit einem Schließhahnblock mit oder ohne integrierten Druckminderer ausgestattet ist, eine Schutzhaube (1) umfassend, die um mindestens einen Teil des Schließhahnblocks herum angeordnet ist, wobei der Schließhahnblock einen auf der Rückseite angeordneten Füllstutzen (15) umfasst und zum Einleiten des Gases in den Behälter (10) dient und wobei die Schutzhaube (1) Folgendes umfasst:
- einen Haubenkörper (2), der eine Schutzhülle bildet, die ein zur Aufnahme des Schließhahnblocks gestaltetes Innenvolumen ganz oder teilweise definiert, und
- eine Einrastvorrichtung (3), die zwischen mehreren Winkellagen schwenkbar ist, umfassend:
. eine Ruheposition, in der die Einrastvorrichtung (3) vollständig eingeklappt ist, und
. eine Einrastposition, in der die Einrastvorrichtung (3) vollständig ausgeklappt ist,
und wobei
- die Einrastvorrichtung (3) eine dreidimensionale Struktur (3, 6a, 6b, 11a, 11b) umfasst, die zwei Arme (6a, 6b) umfasst, die jeweils eine Kerbe (11a, 11b) zur Befestigung an einer Halterung tragen, wobei die dreidimensionale Struktur (3, 6a, 6b, 11a, 11b) eine Innenseite (13) umfasst, die so geformt ist, dass sie sich dem Außenprofil (14) des Körpers (2) der Haube (1) anpasst, wenn die Einrastvorrichtung (3) in der Ruheposition ist, und
- die dreidimensionale Struktur (3, 6a, 6b, 11a, 11b) gegenüber dem Füllstutzen (15) angeordnet ist, wenn die dreidimensionale Struktur (3, 6a, 6b, 11a, 11b) in der Ruheposition ist, und gegen den Körper (2) der Haube (1) eingeklappt ist.

2. Behälter nach dem vorstehenden Anspruch, **dadurch gekennzeichnet, dass** die Einrastvorrichtung (3) um eine AA-Achse schwenkbar befestigt ist, die sich auf der Rückseite (2b) des Körpers (2) der Haube (1) befindet.

3. Behälter nach einem der vorstehenden Ansprüche, **dadurch gekennzeichnet, dass** die Rückseite (2b) und/oder die lateralen Seiten des Körpers (2) der Haube (1) so geformt sind, um ein Außenprofil aufzuweisen, das mit der Innenseite (13) der dreidimensionalen Struktur (3, 6a, 6b, 11a, 11b) komplementär ist.

4. Behälter nach einem der vorstehenden Ansprüche, **dadurch gekennzeichnet, dass** die Rückseite (2b) und/oder die lateralen Seiten des Körpers (2) der Haube (1) mindestens eine Prägung (14) in der Außenhülle des Körpers (2) aufweisen, in der mindestens ein Teil der dreidimensionalen Struktur (3, 6a, 6b, 11a, 11b) durch Anpassen an das Außenprofil der mindestens einen Prägung (14) untergebracht ist.

5. Behälter nach Anspruch 1, **dadurch gekennzeichnet, dass** die dreidimensionale Struktur (3, 6a, 6b, 11a, 11b) mindestens eine gekrümmte Innenseite (13) umfasst.

6. Behälter nach Anspruch 1, **dadurch gekennzeichnet, dass** die Einrastvorrichtung (3) zwischen der Ruheposition und der Einrastposition um einen Winkel (α) kleiner als 180°, typischerweise zwischen 90° und 170°, schwenkt.

7. Behälter nach Anspruch 1, **dadurch gekennzeichnet, dass** er mindestens eine Haltestütze (5) umfasst, die fest mit dem Haubenkörper (2) verbunden ist und einen Tragegriff (4) trägt.

8. Behälter nach Anspruch 1, **dadurch gekennzeichnet, dass** der Körper (4) der Haube (1) und die dreidimensionale Struktur (3, 6a, 6b, 11a, 11b) aus Polymermaterial bestehen.

9. Behälter nach Anspruch 1, **dadurch gekennzeichnet, dass** die beiden Arme (6a, 6b) durch mindestens ein Verbindungselement (6c) miteinander verbunden sind, vorzugsweise die beiden Arme (6a, 6b) und das mindestens eine Verbindungselement (6c) aus einem Stück, insbesondere durch Formguss, geformt sind.

10. Behälter nach Anspruch 1, **dadurch gekennzeichnet, dass** er ein Arretiersystem (16) umfasst, das das Arretieren und Halten der Einrastvorrichtung (3) in der Ruheposition ermöglicht.

11. Behälter nach Anspruch 1, **dadurch gekennzeichnet, dass** die dreidimensionale Struktur (3, 6a, 6b, 11a, 11b) so geformt ist, dass sie einen Schutz des Füllstutzens (15) ermöglicht, wobei eine Schutzabdeckung in der Öffnung oder dem Abstand (12) zwischen den beiden Armen (6a, 6b) der dreidimensionalen Struktur (3, 6a, 6b, 11a, 11b) angeordnet ist und den Füllstutzen (15) abdeckt, wenn die dreidimensionale Struktur (3, 6a, 6b, 11a, 11b) in der Ruheposition eingeklappt ist.

12. Behälter nach Anspruch 1, **dadurch gekennzeichnet, dass** er ausgewählt ist aus Gasflaschen, vorzugsweise einer Gasflasche (10), die eine Größe zwischen 10 und 150 cm aufweist, die 0,5 bis 20 Liter (Wasseräquivalentkapazität) enthält.

13. Verwendung eines Gasbehälters nach einem der vorstehenden Ansprüche zur Verteilung eines Gases oder Gasgemisches.

14. Verwendung nach Anspruch 13, **dadurch gekennzeichnet, dass** das Gas oder Gasgemisch ausgewählt ist aus Sauerstoff, Luft, N₂O/O₂, He/O₂ und NO/Stickstoff.

## Claims

1. Gas container (10) equipped with a tap unit with or without an integrated expander, comprising a protective cowling (1) arranged around at least one part of said tap unit, said tap unit comprising a filling connector (15) situated in the rear face and being used to introduce gas into the container (10) and said protective cowling (1) comprising:
- a cowling body (2) forming a protective shell delimiting an inner volume designed to receive all or part of the tap unit, and
- a hanging device (3) pivoting between several angular positions comprising:
. a rest position, wherein the hanging device (3) is totally folded and
. a fastening position, wherein the hanging device (3) is totally unfolded, and
wherein
the hanging device (3) comprising a three-dimensional structure (3, 6a, 6b, 11a, 11b) comprising two arms (6a, 6b) each carrying a support-fixing notch (11a, 11b), said three-dimensional structure (3, 6a, 6b, 11a, 11b) comprising an inner face (13) shaped to mould the outer profile (14) of the cowling (1) body (2), when the hanging device (3) is in the rest position, and
- the three-dimensional structure (3, 6a, 6b, 11a, 11b) is positioned facing the filling connector (15), when said three-dimensional structure (3, 6a, 6b, 11a, 11b) is in the rest position and folded against the body (2) of the cowling (1).

2. Container according to the preceding claim, **characterised in that** the hanging device (3) is secured so as to pivot about an axis AA situated on the rear face (2b) of the body (2) of the cowling (1).

3. Container according to one of the preceding claims, **characterised in that** the rear face (2b) and/or the side faces of the cowling (1) body (2) are shaped to have a complementary outer profile of the inner face (13) of the three-dimensional structure (3, 6a, 6b, 11a, 11b).

4. Container according to one of the preceding claims, **characterised in that** the rear face (2b) and/or the side faces of the cowling (1) body (2) having at least one footprint (14) made in the outer shell of the body (2), where at least one part of the three-dimensional structure (3, 6a, 6b, 11a, 11b) is housed by moulding the outer profile of said at least one footprint (14).

5. Container according to claim 1, **characterised in that** the three-dimensional structure (3, 6a, 6b, 11a, 11b) comprises at least one curved inner face (13).

6. Container according to claim 1, **characterised in that** the fastening device (3) pivots between the rest position and the fastening position of an angle (α) less than 180°, typically between 90° and 170°.

7. Container according to claim 1, **characterised in that** it comprises at least one support strut (5) secured to the cowling body (2) and carrying a handle (4).

8. Container according to claim 1, **characterised in that** the body (4) of the cowling (1) and the three-dimensional structure (3, 6a, 6b, 11a, 11b) are formed of polymer material.

9. Container according to claim 1, **characterised in that** the two arms (6a, 6b) are connected to one another by at least one sealing element (6c), preferably the two arms (6a, 6b) and said at least one sealing element (6c) are formed from one single part, in particular by moulding.

10. Container according to claim 1, **characterised in that** it comprises a blocking system (16) making it possible to block and hold the hanging device (3) in the rest position.

11. Container according to claim 1, **characterised in that** the three-dimensional structure (3, 6a, 6b, 11a, 11b) is shaped so as to make it possible for a protection of the filling connector (15), a protective cover being situated in the opening or space (12) between the two arms (6a, 6b) of the three-dimensional structure (3, 6a, 6b, 11a, 11b) and covering the filling connector (15), when the three-dimensional structure (3, 6a, 6b, 11a, 11b) is folded in the rest position.

12. Container according to claim 1, **characterised in that** it is selected from among gas cylinders, preferably a gas cylinder (10) of a size of between 10 and 150cm, and containing 0.5 to 20 litres (water-equivalent volume).

13. Use of a gas container according to one of the preceding claims to distribute a gas or gaseous mixture.

14. Use according to claim 13, **characterised in that** the gas or gaseous mixture is selected from among oxygen, air, N₂O/O₂, He/O₂ and NO/nitrogen.
